Europäisches Patentamt

European Patent Office

Office européen des brevets

(11) Veröffentlichungsnummer: **0 280 975 B1**

(19)

(12) **EUROPÄISCHE PATENTSCHRIFT**

(45) Veröffentlichungstag der Patentschrift: **08.07.92**

(21) Anmeldenummer: **88102502.7**

(22) Anmeldetag: **20.02.88**

(51) Int. Cl.⁵: **C07C 223/06**, C07D 319/08, C07C 251/00, C07C 217/76

(54) **Verfahren zur Herstellung von substituierten Benzaldehyden und dabei anfallende Zwischenprodukte.**

(30) Priorität: **03.03.87 CH 798/87**
**16.12.87 CH 4896/87**

(43) Veröffentlichungstag der Anmeldung:
**07.09.88 Patentblatt 88/36**

(45) Bekanntmachung des Hinweises auf die Patenterteilung:
**08.07.92 Patentblatt 92/28**

(84) Benannte Vertragsstaaten:
**AT BE CH DE FR GB IT LI NL**

(56) Entgegenhaltungen:
**EP-A- 0 200 947**
**DD-A- 94 177**
**US-A- 4 627 939**

**CHEMICAL ABSTRACTS, Band 71, Nr. 20, 17. November 1969, Columbus, Ohio, USA; W. BROSER et al: "Substituent effects in triphenylmethane dyes.I.Syntheses, spectroscopic characterization, equilibrium, and kinetic measurements of m-methoxylated p-fuchsines", Seite 72, Spalte 2, Zusammenfassung Nr. 92623x**

(73) Patentinhaber: **F. HOFFMANN-LA ROCHE AG**
**Postfach 3255**
**CH-4002 Basel(CH)**

(72) Erfinder: **Kägi, Dieter, Dr.**
**Grenzacherstrasse 477**
**verstorben(CH)**
Erfinder: **Schlageter, Markus, Dr.**
**Nussbaumweg 24**
**CH-4103 Bottmingen(CH)**
Erfinder: **Widmer, Erich, Dr.**
**Mittelweg 47**
**CH-4142 Münchenstein(CH)**

(74) Vertreter: **Grossner, Lutz, Dr. et al**
**Grenzacher Strasse 124 Postfach 3255**
**CH-4002 Basel(CH)**

CHEMICAL ABSTRACTS, Band 106, Nr. 19, 11. Mai 1987, Columbus, Ohio, USA; M. G. SAGI-NASHIVILI et al: "Synthesis of p-aminophenyl derivatives of 1,3-dioxacycloalkanes", S. 681, Spalte 2, Zu-sammenfassung Nr. 156368u

HOUBEN WEYL, "Methoden der organischen Chemie", 4. Auflage, Band VI, Teil 3: "Sauerstoffverbindungen I", 1965, Georg-Thieme-Verlag, Stuttgart, S. 75-79

**Beschreibung**

Die Erfindung betrifft ein Verfahren zur Herstellung von substituierten Benzaldehyden und in diesem Verfahren verwendete neue Verbindungen. Insbesondere betrifft die Erfindung ein Verfahren zur Herstellung von 4-Dimethylamino-3,5-di-(niederalkoxy)benzaldehyden, dadurch gekennzeichnet, dass man

a) 4-Amino-3,5-dibrombenzaldehyd oder ein cyclisches Acetal oder das Oxim davon mit einem Alkalimetall-nieder-alkoxid in Gegenwart von Kupfer und einem protonierbaren organischen Lösungsmittel umsetzt, das Reaktionsprodukt mit einem Methylierungsmittel behandelt und eine im Reaktionsprodukt enthaltene Acetal- oder Oximgruppe hydrolysiert; oder

b) ein 4-Brom-2,6-di-(nieder-alkoxy)-N,N-dimethylanilin mit einem Metallierungsmittel und danach mit einem Formylierungsmittel umsetzt.

Eine niedere Alkoxygruppe und ein niederes Alkanol sind vorzugsweise geradkettig und enthalten bis zu 6 C-Atome. Eine bevorzugte Alkoxygruppe ist Methoxy.

Die Umsetzung des 4-Amino-3,5-dibrombenzaldehyds oder eines cyclischen Acetals davon oder des Oxims mit einem Alkalialkoholat wird zweckmässig bei erhöhter Temperatur, vorzugsweise bei Temperaturen von etwa 80°C vorgenommen. Das Alkalialkoholat wird zweckmässig im Ueberschuss, z.B. einem 15-20-fachen molaren Ueberschuss eingesetzt. Kupfer kann als Metallpulver eingesetzt werden, vorzugsweise verwendet man jedoch eine Cu(I)-Verbindung wie $Cu_2O$ oder ein Cu-(I)-Salz, insbesondere ein Halogenid wie Kupfer-(I)-jodid. Das Cu-(I)-Salz kann in katalytischen Mengen, z.B. in Mengen von 2-3 Mol-% eingesetzt werden, $Cu_2O$ wird zweckmässig in äquimolarer Menge eingesetzt. Als protonierbare organische Lösungsmittel kommen insbesondere Dimethylformamid oder auch stickstoffhaltige organische Lösungsmittel wie Pyridin oder Collidin in Betracht. In einer bevorzugten Ausführungsform verwendet man ein Lösungsmittelgemisch, in dem ein Lösungsmittel wie Dimethylformamid überwiegt. Vorzugsweise geht man von einem cyclischen Acetal des 4-Amino-3,5-dibrombenzaldehyd aus. Beispiele solcher cyclischen Acetale sind das Aethylen-, das 2,3-Butylen- und das 2,2-Dimethyl-1,3-propylenacetal. Ein bevorzugtes Ausgangs- material ist das 4-(5,5-Dimethyl-1,3-dioxan-2-yl)-2,6-dibromanilin.

Als Methylierungsmittel kommen Reagenzien wie vorzugsweise Formaldehyd/Ameisensäure in Betracht, jedoch können auch andere Methylierungsmittel, z.B. Dimethylsulfat, angewandt werden.

Als Metallierungsmittel kommen in der Verfahrensvariante b) organische Lithiumverbindungen, wie Lithiumalkyle, z.B. Methyl- oder Butyl-Li; oder Phenyl-lithium; oder Magnesiumverbindungen in Betracht. Beispiele von Formylierungsmitteln sind Dimethylformamid, Orthoameisensäureester und N-Formylmorpholin. Die Metallierungsreaktion kann unter für die Herstellung von Lithium- oder Magnesium-organischen bzw. Grignard-Verbindungen an sich bekannten Bedingungen erfolgen. In einer bevorzugten Ausführungsform setzt man das 4-Brom-2,6-di-nieder-alkoxy)-N,N-dimethylanilin mit Butyllithium in Hexan bei etwa -10°C um. Als Formylierungsmittel ist Dimethylformamid bevorzugt.

Ein weiterer Aspekt der Erfindung betrifft ein Verfahren zur Herstellung der cyclischen Acetale des 4-Amino-3,5-dibrom-benzaldehyds, insbesondere des 4-(5,5-Dimethyl-m-dioxan-2-yl)-2,6-dibromanilins, und des 3,5-Dibrom-4-amino-benzaldehydoxims, welches dadurch gekennzeichnet ist, dass man ein cyclisches Acetal von p-Nitrobenzaldehyd, insbesondere das 2-(4-Nitrophenyl)-5,5-dimethyl-1,3-dioxan, oder das 4-Nitrobenzaldehydoxim zu einem cyclischen Acetal von p-Aminobenzaldehyd, insbesondere p-(5,5-Dimethyl-1,3-dioxan-2-yl)anilin, bzw. 4-Amino-benzaldehydoxim reduziert und letztere bromiert, oder dass man p-Nitrotoluol mit einem nieder-Alkylnitrit in Gegenwart einer Base zu p-Aminobenzaldehydoxim umsetzt und dieses bromiert. Die Reduktion des p-Nitrobenzaldehydacetals zu einem p-Amino-benzaldehyacetal bzw. des 4-Nitrobenzaldehydoxims zu p-Aminobenzaldehydoxim wird zweckmässig durch katalytische Hydrierung, z.B. in Gegenwart von Pt- oder Pd-Katalysatoren; oder mit Raney-Ni vorgenommen. Bei der Umsetzung von p-Nitrotoluol mit einem nieder-Alkylnitrit wird vorzugsweise Isoamylnitrit, als Base vorzugsweise ein Alkalialkoholat, z.B. Natriumäthylat in Aethanol verwendet. Die Bromierung kann mit elementarem Brom in Gegenwart eines Protonenakzeptors, wie Butylenoxid, vorgenommen werden.

Ein weiterer Aspekt der Erfindung betrifft ein Verfahren zur Herstellung von 4-Brom-2,6-di-(nieder-alkoxy)-N,N-dimethylanilinen,welches dadurch gekennzeichnet ist, dass man ein 2,6-di-(nieder-Alkoxy)anilin zu einem 4-Brom-2,6-di-(nieder-alkoxy)anilin bromiert und letzteres methyliert. Die Bromierung kann durch Behandlung mit elementarem Brom in einem Lösungsmittel, z.B. einem chlorierten Kohlenwasserstoff, wie Dichlormethan, vorgenommen werden. Die Methylierung kann durch Behandlung mit einem Methylierungsmittel, wie Dimethylsulfat, vorgenommen werden.

Die in den oben beschriebenen Reaktionen erhaltenen Verbindungen

p-(5,5-Dimethyl-1,3-dioxan-2-yl)anilin,
4-(5,5-Dimethyl-1,3-dioxan-2-yl)-2,6-dibromanilin,
4-Brom-2,6-dimethoxyanilin,
4-Brom-2,6-dimethoxy-N,N-dimethylanilin,

4-Amino-3,5-dibrombenzaldehydoxim und
4-Amino-3,5-dimethoxybenzaldehydoxim
sind neu und ebenfalls Gegenstand der Erfindung.

Die erfindungsgemäss erhältlichen 4-Dimethylamino-3,5-di-(nieder-alkoxy)benzaldehyde sind Zwischenprodukte für die Herstellung von als Pharmazeutika wertvollen Benzylpyrimidinen, vgl. z.B. die DE-Offenlegungsschrift Nr. 2 443 682. Die bisher bekannten Herstellungsverfahren für diese Benzylpyrimidine sind kostspielig. Die vorliegende Erfindung ermöglicht es nun, diese Verbindungen wesentlich kostengünstiger als bisher herzustellen. Dies wird dadurch erreicht, dass die als Schlüssel-Zwischenprodukte benötigten Benzaldehyd-Derivate aus billigen Rohstoffen unter Verwendung einfacher Reaktionen in hoher Ausbeute zugänglich gemacht werden.

Die Erfindung wird durch die nachstehenden Beispiele weiter erläutert. Die Temperaturen sind in Celsiusgraden angegeben.

## Beispiel 1

Ein Gemisch von 765 g 4-Nitrobenzaldehyd, 537,5 g 2,2-Dimethyl-1,3-propandiol, 0,58 g p-Toluolsulfonsäuremonohydrat und 2530 ml Toluol wurde während 15 Stunden zum Rückfluss erhitzt, bis sich ca. 85 ml Wasser abgeschieden hatten und kein Edukt mehr nachgewiesen werden konnte. Das Reaktionsgemisch wurde abgekühlt, und mit 500 ml Wasser gewaschen. Die wässrige Phase wurde mit 1500 ml tert. Butylmethyläther extrahiert, der Extrakt mit 1000 ml gesättigter, wässriger Kochsalzlösung gewaschen und die organischen Phasen über Natriumsulfat getrocknet. Das Lösungsmittel wurde bei 50°/15 mbar abdestilliert und der Rückstand bei 50°/0,01 mbar getrocknet. Man erhielt 1195,4 g (99%) beiges Kristallisat vom Schmelzpunkt 81-84°. Die gaschromatographisch ermittelte Reinheit betrug 98,5%. Dieses Rohprodukt wurde aus n-Hexan umkristallisiert, wobei 1128 g (94%) 2-(4-Nitrophenyl)-5,5-dimethyl-1,3-dioxan als hellgelbes Kristallisat vom Schmelzpunkt 83-85° erhalten wurden. Die gaschromatographisch ermittelte Reinheit betrug 100%.

## Beispiel 2

Ein Gemisch bestehend aus 1125 g 2-(4-Nitrophenyl)-5,5-dimethyl-1,3-dioxan, 112,5 g Platin auf Kohle und 11250 ml Tetrahydrofuran wurde bei 25° bei einem $H_2$-Druck von 10 bar während 8 Stunden hydriert. Der Ansatz wurde wie folgt aufgearbeitet:

Alle Operationen wurden unter $N_2$-Begasung durchgeführt. Der Katalysator wurde durch Filtration über ein Dicaliterpolster abgetrennt, das Filtrat bei 60°/15 mbar vom Lösungsmittel befreit und der Rückstand bei 60°/0,1 mbar getrocknet. Man erhielt 945,2 g (96,2%) 5,5-Dimethyl-1,3-dioxan-2-yl)anilin als beiges Kristallisat vom Schmelzpunkt 102-104°.

## Beispiel 3

Zu einer Lösung von 10,36 g p-(5,5-Dimethyl-1,3-dioxan-2-yl)anilin, 14,42 g Butylenoxid in 200 ml Dichlormethan wurden unter Eiskühlung unter Rühren innert 1,5 Stunden eine auf 0-5° vorgekühlte Lösung von 16,78 g Brom in 500 ml Dichlormethan getropft. Die Reaktion wurde über Nacht durch Rühren bei Raumtemperatur vervollständigt. Das Reaktionsgemisch wurde unter Rühren bei Raumtemperatur nacheinander mit 7,5 ml 28% wässriger Natriumhydroxidlösung bis zum pH von ca. 12 und innert 15 Minuten mit 130 ml 10% wässriger Natriumthiosulfatlösung versetzt, die organische Phase abgetrennt und die wässrige Phase nacheinander mit 2 Portionen zu 250 ml Dichlormethan nachextrahiert. Die vereinigten organischen Phasen wurden über 30 g Natriumsulfat getrocknet, das Lösungsmittel bei 50°/15 mbar abdestilliert und der Rückstand bei 50°/0,01 mbar getrocknet. Man erhielt 17,4 g (95,3%) beige Kristalle vom Schmelzpunkt 154-156°. Die gaschromatographisch ermittelte Reinheit betrug 88,3%. Das Rohprodukt wurde aus Aethanol umkristallisiert und lieferte 12,8 g (70%) 4-(5,5-Dimethyl-1,3-dioxan-2-yl)-2,6-dibromanlin als weisse Kristalle vom Schmelzpunkt 156-157°. Die gaschromatographisch ermittelte Reinheit betrug 100%.

## Beispiel 4

Aus einer Lösung von 1235 ml 30% methanolischer Natriummethylatlösung und 450 ml Dimethylformamid wurden innert 45 Minuten unter Rühren und Stickstoffbegasung bei 103-118° (Badtemperatur 120-140°) 630 ml Lösungsmittelgemisch abdestilliert. Dabei trat gegen Ende des Abdestillierens eine weisse Fällung (Na-Methylat) ein. Nun wurde die Temperatur auf 80±2° abgesenkt (Badtemperatur 90-92°) und das Reaktionsgemisch in einer Portion mit 16,6 g Kupfer-(I)-jodid (fein gepulvert) versetzt. Innert 15 Minuten wurden in Portionen zu ca. 5 g, 121,6 g 4-(5,5-Dimethyl-1,3-dioxan-2-yl)-2,6-dibrom-anilin zugefügt. Unter gutem Rühren wurde die Rekation 3,5 Stunden bei 80±2° fortgesetzt, bis sich das Ausgangsmaterial nicht mehr nachweisen liess. Das auf Raumtemperatur abgekühlte Reaktionsgemisch wurde unter Rühren und Kühlen auf 1050 ml Wasser gegossen, und 4 Stunden auf 60° erwärmt. Das auf Raumtemperatur gekühlte Gemisch wurde 15 Minuten mit 5 g Aktivkohle und 2200 ml Toluol verrührt und über Dicalite genutscht. Die wässrige Phase wurde zweimal mit 900 ml Toluol extrahiert. Das Lösungsmittel wurde bei 60°/15 mbar abdestilliert und der Rückstand bei 60°/0,01 mbar getrocknet. Der Rückstand (87,6 g bräunliches Oel) wurde mit 400 g 98% Ameisensäure und 58 g 37-40% wässriger Formaldehydlösung während 180 Minuten bei 89-94° (Badtemperatur 92-102°) zum Rückfluss erhitzt. Anschliessend wurden 23 ml 37% Salzsäure zugesetzt und dass Gemisch weitere 2 Stunden zum Rückfluss erhitzt. Das Reaktionsgemisch wurde bei 55-60°/15 mbar vom Formaldehyd und der Ameisensäure befreit. Der Rückstand wurde mit 1140 ml Eiswasser verdünnt, unter Rühren und Kühlen bei 10-15° bis zum pH von ca. 8,5 mit 81 ml 28% wässriger Natronlauge versetzt und mit dreimal 800 ml Toluol extrahiert. Die organische Phase wurde mit 240 ml gesättigter wässriger Kochsalzlösung gewaschen, über Natriumsulfat getrocknet, das Lösungsmittel bei 60°/15 mbar abdestilliert und der Rückstand bei 60°/0,1 mbar getrocknet. Man erhielt als Rückstand 67,1 g gelbes Oel, das im Hochvakuum über eine 25 cm Vigreux-Kolonne destilliert wurde. Bei 110-120°/0,1 mbar wurden 54,4 g 4-(Dimethylamino)-3,5-dimethoxybenzaldehyd als hellgelbes Oel erhalten, das beim Stehenlassen durchkristallisierte. Die gaschromatographisch ermittelte Reinheit betrug 98% (Smp. 46-47°).

Beispiel 5

Zu einer Lösung von 76,6 g 2,6-Dimethoxyanilin in 8 l Dichlormethan wurde bei ca. 1° eine Lösung von 78,9 g Brom in 1 l Dichlormethan so getropft, dass das Reagens zu Beginn relativ schnell (750 ml in 2 Stunden) danach langsamer zugegeben wurde, um Ueberbromierung zu vermeiden. Nach etwa 6 Stunden war alles Reagens zugesetzt. Danach wurde 25 Minuten nachgerührt. Das Reaktionsgemisch wurde mit 2 l 1N Natronlauge und zweimal mit je 1,25 l Wasser gewaschen, die Waschlösung mit 2 Portionen zu 1,25 l Dichlormethan re-extrahiert und die vereinigten organischen Phasen über Natriumsulfat getrocknet. Das Lösungsmittel wurde bei einer Badtemperatur von 50° eingedampft und der Rückstand getrocknet. Es resultierten 114,2 g 4-Brom-2,6-dimethoxyanilin als amorphe, braune Masse. Die gaschromatographisch bestimmte Reinheit betrug 97%. Dieses Rohprodukt wurde in 300 ml Toluol gelöst und durch Filtration über 1 kg Kieselgel 60 und Elution mit 16 l Toluol gereinigt. Das Eluat wurde bei einer Badtemperatur von 80° eingedampft und der Rückstand getrocknet. Es resultierten 94,4 g 4-Brom-2,6-dimethoxyanilin als schwach beige Kristalle vom Schmelzpunkt 71-73° und einer gaschromatographisch ermittelten Reinheit von 99%.

Beispiel 6

Zu einer Suspension von 136 g Natriumbicarbonat in 200 ml Wasser wurden unter starkem Rühren 95,2 g 4-Brom-2,6-dimethoxyanilin gegeben und die Suspension mit einem Eisbad auf 10° abgekühlt. Dann wurden in einer Portion 181 g Dimethylsulfat zugesetzt und das heterogene Gemisch während 2,5 Stunden bei 10° stark gerührt, bis keine Gasentwicklung und kein Edukt mehr feststellbar war. Es wurde bis zum Erreichen von Raumtemperatur nachgerührt, mit 90 ml 25% wässriger Ammoniaklösung versetzt und eine weitere Stunde gerührt. Das Reaktionsgemisch wurde mit 360 ml Dichlormethan extrahiert, die organische Phase mit 450 ml Salzsäurelösung behandelt und die wässrige Phase mit 180 ml Dichlormethan gewaschen. Zur salzsauren, wässrigen Phase wurden unter Rühren und Kühlen ~68 ml 28% wässrige Natriumhydroxidlösung getropft, bis pH 8,5 erreicht war und das Produkt auszukristallisieren begann. Zur Vervollständigung der Kristallisation wurde die Suspension auf 0° gekühlt. Die weissen Kristalle wurden abgenutscht, mit 200 ml Eiswasser neutral gewaschen, 3 Stunden bei 50°/15 mbar und über Nacht bei 50° im Hochvakuum getrocknet. Es resultierten 100,4 g 4-Brom-2,6-dimethoxy-N,N-dimethylanilin als weisse Kristalle vom Schmelzpunkt 90-91°. Die gaschromatographisch ermittelte Reinheit betrug 100%.

Beispiel 7

13 g 4-Brom-2,6-dimethoxy-N,N-dimethylanilin wurden unter Stickstoff in 165 ml Aether gelöst. Zu der auf -10° abgekühlten Lösung wurde innert 20 Minuten 50 g Butyllithium in Hexan getropft und die entstandene weisse Suspension 30 Minuten bei -10° weitergerührt. Unter guter Durchmischung wurde bei gleicher Temperatur innert 1 Stunde eine Lösung von 28,6 g Dimethylformamid in 250 ml Aether zugetropft und das Gemisch 4 Stunden bei Raumtemperatur nachreagieren gelassen. Anschliessend wurde das Gemisch auf 20 g konz. Salzsäure und 20 g Eis gegossen und unter gutem Rühren während 1 Stunde hydrolysiert (pH ca. 1,2). Bei 5-10° (Kühlung durch Eis) wurde die wässrige Phase durch Zugabe von ca. 20 ml 28% Natronlauge auf pH 8,5 gebracht. Die wässrige Phase wurde mit 480 ml Aether extrahiert; die Aetherphase mit 120 ml halbgesättigter Kochsalzlösung gewaschen, über Natriumsulfat getrocknet und abfiltriert, das Filtrat im Wasserstrahlvakuum bei einer Badtemperatur von 50° eingedampft und der Rückstand im Hochvakuum getrocknet. Es resultierten 10,5 g 4-(Dimethylamino)-3,5-dimethoxybenzaldehyd als Oel, das im Kühlschrank zu gelben Kristallen erstarrte. Die gaschromatographisch ermittelte Reinheit betrug 88,5%.

### Beispiel 8

13,6 g 4-Amino-benzaldehydoxim wurden in 300 ml Eisessig unter Rühren gelöst. Die Temperatur wurde auf 0-2° abgesenkt und aus dem Tropftrichter innert 20 Minuten eine Lösung von 35,2 g Brom in 100 ml Eisessig so schnell zugetropft, dass die Temperatur den Bereich von 15-20° nicht überschritt. Das Reaktionsgemisch wurde sofort auf 1000 ml 1:1-Gemisch Eis/Wasser gegossen und 30 Minuten durchgerührt. Die ausgefallenen Kristalle wurden abgetrennt und mit zwei Portionen zu 250 ml, d.h. total mit 500 ml Eiswasser aufgeschlämmt und trocken gesaugt. Die Kristalle wurden über Nacht bei Raumtemperatur über Kaliumhydroxyd im Wasserstrahlvakuum bis zur Gewichtskonstanz getrocknet. Man erhielt 20 g 3,5-Dibrom-4-aminobenzaldehydoximals ockerfarbige Kristalle vom Schmelzpunkt 144-145°. Die gaschromatographisch ermittelte Reinheit betrug 83%.

### Beispiel 9

Aus einem Gemisch von 1527 ml 30% methanolische Natriummethylatlösung und 562 ml Dimethylformamid wurden 810 ml Lösungsmittelgemisch abdestilliert, bis Natriummethylat auszufallen begann. Nach dem Absenken der Temperatur auf 80±2° (Bad = 82°) wurden in einer Portion 21,38 g Kupfer(I)jodid als fein zerriebenes Pulver zugesetzt, wobei das Gemisch eine tiefblaue Farbe annahm, die nach 15 Minuten Rühren nach violett umschlug. Innert 5 Minuten wurden in Portionen zu ca. 1,5 g total 118 g 3,5-Dibrom-4-aminobenzaldehydoxim beigefügt und das Gemisch während 2 Stunden bei 80° gerührt, bis sich weder Edukt noch Monomethoxyzwischenprokukt nachweisen liess. Das auf 60° abgekühlte Reaktionsgemisch wurde innert 15 Minuten zu 2,5 kg Eis/Wasser = 1:1 gegeben, das Gemisch auf 60° gebracht und unter Rühren während 8 Stunden belassen. Danach wurde das Gemisch zu 1000 ml eisgekühlter konzentrierter wässriger Salzsäure unter Rühren zufliessen gelassen. Die Neutralteile wurden mit zwei Portionen zu 1000 ml, d.h. total mit 2000 ml Toluol extrahiert. Unter Zugabe von Eisstücken wurde die wässrige saure Phase bei Raumtemperatur mit 700 ml 28% wässriger Natronlauge bis zum Erreichen eines pH von ca. 9-10 versetzt und das freigesetzte 4-Amino-3,5-dimethoxybenzaldehydoxim mit drei Portionen zu 1000 ml, d.h. total mit 300 ml Toluol extrahiert. Zum Abbinden schlammiger Anteile wurde 20 g Kieselgel 60 dazugegeben und über ein Dicalitpolster genutscht. Das klare, orange zweiphasige Filtrat wurde von der Wasserphase getrennt. Die Toluolphase wurde bie 60°/15 mbar eingeengt und der Rückstand bei 60°/0,1 mbar bis zur Gewischtskonstanz getrocknet. Man erhielt 78,6 g 4-Amino-3,5-dimethoxybenzaldehydoxim als braungelbe feste Masse. Die gaschromatographisch ermittelte Reinheit betrug 66,54%.

### Beispiel 10

78,6 g 4-Amino-3,5-dimethoxybenzaldehydoxim (Rohprodukt aus Beispiel 9) wurden mit 468 ml bzw. 558 g 98% Ameisensäure und mit 80,8 g 37-40% wässriger Formaldehydlösung versetzt und das Gemisch 2 1/4 Stunden zum Rückfluss erhitzt bis kein Edukt mehr vorlag. Anschliessend wurden 31,8 ml bzw. 35,5 g konzentrierter Salzsäure beigefügt und weitere 2 Stunden zum Rückfluss erhitzt. Das auf Raumtemperatur gekühlte Reaktionsgut wurde bei 55°/15 mbar von überschüssiger Formaldehydlösung und Ameisensäure befreit. Der Rückstand wurde mit 1380 g Eiswasser versetzt und unter Zugabe von Eisstücken bei Raumtemperatur mit 62 ml 28% wässriger Natronlauge versetzt, bis ein pH von 8,5 erreicht war. Der freigesetzte 4-Dimethylamino-3,5-dimethoxybenzaldehyd wurde mit drei Portionen zu 1000 ml, d.h. total mit 3000 ml Toluol extrahiert. Die Toluolphasen wurden nacheinander mit 300 ml gesättigter wässriger Kochsalzlösung gewaschen. Die wässrigen Phasen wurden verworfen, die Toluolphasen vereinigt und über 200 g Natriumsulfat getrocknet. Das Trocknungsmittel wurde abgenutscht und mit zwei Portionen zu 100 ml, d.h. total mit 200 ml Toluol aufgeschlämmt und trocken gesaugt. Das Lösungsmittel des Filtrates wurde bei 60°/15 mbar abdestilliert und der Rückstand bei 60°/0,1 mbar bis zur Gewichtskonstanz getrocknet. Man erhielt 54,2 g 4-Dimethylamino-3,5-dimethoxybenzaldehyd als gelbbraunes Oel mit einer gaschromatographisch ermittelten Reinheit von 96%.

### Beispiel 11

14,3 g Kupfer(I)-oxid wurden unter Argon mit 370 ml Natriummethylatlösung (2 Mol) 10 Minuten unter Rühren am Rückfluss erhitzt und innert 10 Minuten mit einer Lösung von 36,5 g 4-(5,5-Dimethyl-1,3-dioxan-2-yl)-2,6-dibromanilin in 200 ml N,N-Dimethylformamid versetzt. Danach wurden 175 ml Lösungsmittel abdestilliert. Nach 90 Minuten Rühren bei 80-108° wurde das Reaktionsgemisch abgekühlt, mit ca. 200 ml Methylenchlorid in einen 2 Liter-Rundkolben gespült und am Rotationsverdampfer bei max. 6°/30 mbar eingeengt. Der braun-rote, feste Rückstand wurde in 750 ml 3N Natronlauge aufgenommen, die Suspension 60 Minuten am Rückfluss gerührt, auf 60° abgekühlt, mit 300 ml Aethylacetat versetzt, 10 Minuten gerührt und über ein Filterhilfsmittel filtriert. Der Filterrückstand wurde dreimal mit je 100 ml Aethylacetat ausgewaschen, die wässerige Phase abgetrennt und noch zweimal mit je 100 ml Aethylacetat nachextrahiert. Die organischen Phasen wurden der Reihe nach mit 250 ml Wasser gewaschen, über 50 g Magnesiumsulfat getrocknet, filtriert, der Filterrückstand mit zweimal je 50 ml Aethylacetat gewaschen und die vereinigten Filtrate am Rotationsverdampfer bei max. 35°/30 mbar eingeengt, wobei 32,1 g 4-(5,5-Dimethyl-1,3-dioxan-2-yl)-2,6-dimethoxyanilin als Rohprodukt erhalten wurden. 31,6 g des so erhaltenen Rohprodukts wurden mit 140 ml Ameisensäure und 22,9 g Formaldehydlösung unter Rühren zum Rückfluss erhitzt. Die braune Lösung wurde auf 0° gekühlt und unter Eiskühlung viermal mit je 100 ml Methylenchlorid gewaschen, die organischen Extrakte einzeln zweimal mit 100 ml 3N Salzsäure extrahiert, die vereinigten wässerigen Phasen wurden im Verlaufe von 2 Stunden bei 0-5° mit 435 ml Natronlauge (28%) auf pH 11 gestellt, die entstandene Emulsion mit 550 ml Methylenchlorid extrahiert, die Extrakte einzeln mit 250 ml kaltem Wasser gewaschen, die organische Phase über 50 g Magnesiumsulfat getrocknet, über eine Glasnutsche mit 40 g Kieselgel filtriert, das Filterbett sechsmal mit je 50 ml Methylenchlorid gewaschen und die vereinigten Filtrate im Rotationsverdampfer bei 30°/30 mbar bis zur Gewichtskonstanz eingedampft. Man erhielt 15,6 g 4-Amino-3,5-dimethoxybenzaldehyd als hellgelbes Oel. Das Filterbett wurde noch dreimal mit je 100 ml Aether gewaschen, die vereinigten Filtrate im Rotationsverdampfer bei 30°/30 mbar bis zur Gewichtskonstanz eingedampft, wobei weitere 1.9 g Reaktionsprodukt erhalten wurden.

## Patentansprüche

1. Verfahren zur Herstellung von 4-Dimethylamino-3,5-di-(nieder-alkoxy)-benzaldehyden, dadurch gekennzeichnet, dass man
    a) 4-Amino-3,5-dibrombenzaldehyd oder ein cyclisches Acetal oder das Oxim davon mit einem Alkalimetall-nieder-alkoxid in Gegenwart von Kupfer und einem protonierbaren organischen Lösungsmittels umsetzt, das Reaktionsprodukt mit einem Methylierungsmittel behandelt und eine im Reaktionsprodukt ethaltene Acetal- oder Oximgruppe hydrolysiert; oder
    b) ein 4-Brom-2,6-di-(nieder-alkoxy)-N,N-dimethylanilin mit einem Metallierungsmittel und danach mit einem Formylierungsmittel umsetzt.

2. Verfahren nach Anspruch 1, dadurch gekennzeichnet, dass man in a) ein Cu(I)-Salz verwendet.

3. Verfahren nach Anspruch 1, dadurch gekennzeichnet, dass man in a) als cyclisches Acetal das 4-(5,5-Dimethyl-1,3-dioxan-2-yl)-2,6-dibromanilin verwendet.

4. Verfahren nach den Ansprüchen 1-3, dadurch gekennzeichnet, dass man das in a) verwendete cyclische Acetal von 4-Amino-3,5-dibrombenzaldehyd durch Reduktion eines cyclischen Acetals von p-Nitrobenzaldehyd zu einem cyclischen Acetal von p-Aminobenzaldehyd und Bromierung der letzteren Verbindung herstellt.

5. Verfahren nach Anspruch 1, dadurch gekennzeichnet, dass man das in a) verwendete 3,5-Dibrom-4-amino-benzaldehydoxim durch Reduktion von 4-Nitrobenzaldehydoxim zu 4-Amino-benzaldehydoxim und Bromierung; oder durch Umsetzung von p-Nitrotoluol mit einem nieder-Alkylnitrit in Gegenwart einer Base und Bromierung des so erhaltenen 4-Aminobenzaldehydoxims herstellt.

6. Verfahren nach Anspruch 1, dadurch gekennzeichnet, dass man das in b) verwendete 4-Brom-2,6-di-(nieder-alkoxy)-N,N-dimethylanilin durch Bromierung eines 2,6-di-(nieder-Alkoxy)-anilins zu einem 4-Brom-2,6-di-(nieder-alkoxy)-anilin und Methylierung der letzteren Verbindung herstellt.

7. Verfahren nach Anspruch 1, dadurch gekennzeichnet, dass man in a) als Alkalimetal-nieder-alkoxid ein Alkalimetallmethoxid verwendet.

8. 4-(5,5-Dimethyl-1,3-dioxan-2-yl)-2,6-dibromanilin.

9. 4-Brom 2,6-dimethoxy-N,N-dimethylanilin.

10. 4-Amino-3,5-dibrombenzaldehydoxim.

11. 4-Amino-3,5-dimethoxybenzaldehydoxim.

## Claims

1. A process for the manufacture of 4-dimethylamino-3,5-di-(lower-alkoxy)-benzaldehydes, characterized by
    a) reacting 4-amino-3,5-dibromobenzaldehyde or a cyclic acetal or the oxime thereof with an alkali metal lower-alkoxide in the presence of copper and a protonizable organic solvent, treating the reaction product with a methylating agent and hydrolyzing an acetal or oxime group present in the reaction product; or

b) reacting a 4-bromo-2,6-di-(lower-alkoxy)-N,N-dimethyl-aniline with a metalating agent and thereafter with a formylating agent.

2. A process according to claim 1, characterized in that a Cu(I) salt is used in a).

3. A process according to claim 1, characterized in that 4-(5,5-dimethyl-1,3-dioxan-2-yl)-2,6-dibromoaniline is used as the cyclic acetal in a).

4. A process according to any one of claims 1-3, characterized in that the cyclic acetal of 4-amino-3,5-dibromo-benzaldehyde used in a) is prepared by reducing a cyclic acetal of p-nitrobenzaldehyde to a cyclic acetal of p-aminobenzaldehyde and brominating the latter compound.

5. A process according to claim 1, characterized in that the 3,5-dibromo-4-amino-benzaldehyde oxime used in a) is prepared by reducing 4-nitrobenzaldehyde oxime to 4-amino-benzaldehyde oxime and brominating the latter; or by reaction p-nitrotoluene with a lower-alkyl nitrite in the presence of a base and brominating the thus-obtained 4-aminobenzaldehyde oxime.

6. A process according to claim 1, characterized in that the 4-bromo-2,6-di-(lower-alkoxy)-N,N-dimethylaniline used in b) is prepared by brominating a 2,6-di-(lower-alkoxy)aniline to give a 4-bromo-2,6-di-(lower-alkoxy)aniline and methylating the latter compound.

7. A process according to claim 1, characterized in that an alkali metal methoxide is used as the alkali metal lower-alkoxide in a).

8. 4-(5,5-Dimethyl-1,3-dioxan-2yl)-2,6-dibromoaniline.

9. 4-Bromo-2,6-dimethoxy-N,N-dimethylaniline.

10. 4-Amino-3,5-dibrombenzaldehyde oxime.

11. 4-Amino-3,5-dimethoxybenzaldehyde oxime.

## Revendications

1. Procédé de préparation de 4-diméthylamino-3,5-di-(alcoxy inférieur)-benzaldéhydes, caractérisé en ce que :

a) on fait réagir le 4-amino-3,5-dibromobenzaldéhyde ou un acétal cyclique de cet aldéhyde ou l'oxime de cet aldéhyde avec un alcoolate inférieur de métal alcalin en présence de cuivre et d'un solvant organique protonisable, on traite le produit de réaction par un agent méthylant et on hydrolyse un groupe acétal ou oxime contenu dans le produit de réaction; ou bien

b) on fait réagir une 4-bromo-2,6-di-(alcoxy inférieur)-N,N-diméthylaniline avec un agent métallisant puis avec un agent formylant.

2. Procédé selon la revendication 1, caractérisé en ce que, dans la variante a), on utilise un sel cuivreux.

3. Procédé selon la revendication 1, caractérisé en ce que, dans la variante a), l'acétal cyclique utilisé est la 4-(5,5-diméthyl-1,3-dioxanne-2-yl)-2,6-dibromaniline.

4. Procédé selon les revendications 1 à 3, caractérisé en ce que, dans la variante a), l'acétal cyclique du 4-amino-3,5-dibromobenzaldéhyde utilisé est préparé par réduction d'un acétal cyclique du p-nitrobenzaldéhyde, donnant un acétal cyclique du p-aminobenzaldéhyde, qu'on soumet à bromuration.

5. Procédé selon la revendication 1, caractérisé en ce que la 3,5-dibromo-4-amino-benzaldoxime utilisée est préparée par réduction de la 4-nitrobenzaldoxime en la 4-aminobenzaldoxime et bromuration; ou bien par réaction du p-nitrotoluène avec un nitrite d'alkyle inférieur en présence d'une base et bromuration de la 4-amino-benzaldoxime ainsi obtenue.

6. Procédé selon la revendication 1, caractérisé en ce que la 4-bromo-2,6-di-(alcoxy inférieur)-N,N-diméthylaniline utilisée dans la variante b) est préparée par bromuration d'une 2,6-di-(alcoxy inférieur)-aniline en une 4-bromo-2,6-di-(alcoxy inférieur)-aniline et méthylation de cette dernière.

7. Procédé selon la revendication 1, caractérisé en ce que l'alcoolate inférieur de métal alcalin utilisé dans la variante a) est un méthylate de métal alcalin.

8. La 4-(5,5-diméthyl-1,3-dioxanne-2-yl)-2,6-dibromaniline.

9. La 4-bromo-2,6-diméthoxy-N,N-diméthylaniline.

10. La 4-anino-3,5-dibromobenzaldoxime.

11. La 4-amino-3,5-diméthoxybenzaldoxime.